# FASCICULE DE BREVET EUROPEEN

(11) **EP 1 605 918 B1**
(45) Date de publication et mention de la délivrance du brevet: **28.09.2016**
(21) Numéro de dépôt: 04742337.1
(22) Date de dépôt: 24.03.2004
(51) Int. Cl.: A61K 9/16, A61K 9/14, A61K 47/12, A61K 47/44, A61K 31/65, A61K 31/167, A61K 31/7048

(54) **SYSTEME GALENIQUE POUR LE TRANSPORT D'ACTIF, PROCEDE DE PREPARATION ET UTILISATION**
GALENISCHES SYSTEM FÜR DIE VERABREICHUNG VON WIRKSTOFFEN, VERFAHREN ZUR HERSTELLUNG UND VERWENDUNG
GALENICAL SYSTEM FOR ACTIVE TRANSPORT, METHOD FOR PREPARATION AND USE

(30) Priorité: 24.03.2003 FR 0303568
(43) Date de publication de la demande: 21.12.2005
(73) Titulaire: Capsugel France, 68000 Colmar (FR)
(72) Inventeur: IOUALALEN, Karim, F-31650 Saint Orens de Gameville (FR); RAYNAL, Rose, Anne, F-31650 Saint Orens de Gameville (FR)
(74) Mandataire: Macchetta, Andrea
(86) Numéro de dépôt international: PCT/FR2004/000729
(87) Numéro de publication internationale: WO 2004/084856

(56) Documents cités:
- EP-A- 0 911 038
- EP-A- 1 197 207
- WO-A-00/76478
- WO-A-00/76481
- WO-A-94/12157
- WO-A-99/13864
- WO-A-99/65448
- GB-A- 1 323 161
- US-A- 4 132 753
- "Lauric acid, Myristic acid, Oleic acid, Palmitic acid" In: "Handbook of Pharmaceutical Excipients, fifth edition", 1 January 2007 (2007-01-01), Pharmaceutical Press, London
- BUSZELLO K ET AL: "The influence of alkali fatty acids on the properties and the stability of parenteral O/W emulsions modified with Solutol HS 15<(>R)", EUROPEAN JOURNAL OF PHARMACEUTICS AND BIOPHARMACEUTICS, ELSEVIER SCIENCE PUBLISHERS B.V., AMSTERDAM, NL, vol. 49, no. 2, 1 March 2000 (2000-03-01), pages 143-149, XP004257148, ISSN: 0939-6411, DOI: 10.1016/S0939-6411(99)00081-8

## Description

La présente invention concerne un nouveau système galénique permettant la protection d'un actif, particulièrement d'un médicament, contre sa dégradation lors du transit dans l'estomac lors d'une absorption par voie orale,

Le système galénique selon l'invention permet en outre le masquage du goût d'un actif éventuellement contenu dans le système galénique, la stabilisation dudit actif, la modulation des propriétés de libération dudit actif, le masquage des effets d'irritabilité mucosale et la toxicité de certains principes actifs.

La voie d'administration thérapeutique la plus simple et la plus pratique reste la voie orale. En France, elle constitue 75 % des prises médicamenteuses (Pharmacie galénique, A. Le Hir - Editions Masson.). Les formes galéniques destinées à la voie orale se présentent essentiellement sous deux formes, liquides et sèches. Elles présentent l'énorme avantage de ne pas nécessiter d'acte médical lors de la prise.

Le pH de l'estomac est compris entre 2 et 6. La nature acide du milieu stomacal peut entraîner la dégradation des actifs contenus dans les compositions ingérées, avant que ceux-ci n'atteignent l'intestin, où ils sont théoriquement absorbés au travers de la muqueuse intestinale pour passer dans la circulation. Un tel effet délétère du transit stomacal peut aller à encontre de l'objectif recherché à savoir l'absorption par l'organisme dudit actif dans sa forme la plus efficace pour l'effet recherché. Cet inconvénient est d'autant plus fort que l'on s'adresse aux compositions pharmaceutiques.

Il existe donc un besoin de véhicule pour les actifs ingérés par la voie orale, capable d'assurer audit actif un transit stomacal au cours duquel ledit actif ne sera pas dégradé. C'est un des buts de la présente invention.

D'autres problèmes sont bien connus avec les formes galéniques destinées à la voie orale, particulièrement les formes galéniques médicamenteuses. Un problème récurrent de ces formes galénique est l'observance.

L'observance est un facteur capital dont dépend directement l'efficacité du traitement thérapeutique. L'observance, on parle aussi de bon usage du médicament, est définie comme étant l'action de suivre un traitement médicamenteux conformément aux indications de la prescription : respect de la durée du traitement, du nombre et des horaires de prises. Pris à une dose ou une fréquence insuffisante, un médicament risque d'être inactif ou peu efficace. Dans le cas d'affections transitoires, une mauvaise observance du traitement ne fait que reculer le moment de la guérison et entraîne des rechutes, parfois responsables de graves complications. Dans le cas de maladies chroniques, une mauvaise observance peut être responsable de dommages irréversibles. Les principales difficultés rencontrées lors de l'administration orale varient en fonction de la présentation.

Pour les formes sèches, cachets, gélules, capsules, les inconvénients sont la déglutition et le goût. Certaines populations comme les personnes âgées, les enfants, certaines personnes présentant des troubles mentaux, doivent s'orienter vers la forme liquide. Pour les formes liquides, la prise est très facile, mais cette forme se heurte toujours à un problème non résolu de masquage du goût et d'instabilité de nombreux principes actifs en phase aqueuse.

C'est également un des objectifs de la présente invention que de proposer un système galénique permettant un masquage du goût efficace.

Enfin, quelle que soit la forme, il existe également des problèmes d'irritabilité, de toxicité mucosale et de gastro-toxicité, rencontrés lors de la prise de certains actifs, particulièrement de médicaments comme les anti-inflammatoires.

C'est aussi un des but de la présente invention que de proposer un système galénique permettant une libération retardée d'un actif, particulièrement afin que celui-ci ne soit pas libéré dans l'estomac lors de l'ingestion. Cette propriété passe par l'obtention d'un système galénique stable en milieu acide, c'est-à-dire résistant à un pH acide.

Comme le rappelle le document PCT/US99/27981 (WO 00/30617), page 2 ligne 4, les méthodes utilisées pour minimiser le mauvais goût sont variées, addition d'édulcorants, addition d'arômes, formulation effervescente et technologies d'enrobage. Seules les techniques d'enrobage tentent de masquer le goût alors que les autres approches essaient d'augmenter l'appétence de la préparation. Ces techniques d'enrobage ont aussi été retenues pour empêcher la libération des actifs gastrotoxique, dans l'estomac.

Les techniques d'enrobage consistent à mettre en place une couche de composés isolants, de polymères ou de mélanges, autour du principe actif pour l'isoler du milieu externe. De nombreux composés polymériques, naturels ou de synthèse ont été utilisés pour la constitution de cette couche externe. On distingue principalement les dérivés de cellulose comme l'hydroxypropylméthylcellulose (HPMC), l'éthylcellulose, les carboxyméthylcelluloses, l'hydroxypropylméthylcellulose phtalate ou les mélanges de ces produits. Cette technique a donné des résultats intéressants pour la modulation de la vitesse de libération et pour la gastroprotection, mais l'homme de l'art sait que le masquage du goût n'est pas satisfaisant et la formulation dans l'eau reste instable dans le temps, ce qui est incompatible avec la préparation de formes aqueuses comme les sirops et les suspensions.

D'autres polymères ont été utilisés comme les dérivés de polyacrylates, les polymères amonio-méthacrylate ou le méthacrylate proposés par la société ROHM, tel que décrit dans le document FR 2795962 et WO 98/47493. De nombreux travaux ont été réalisés avec l'amidon et surtout les polycarbophiles et Carbopol comme décrit dans le brevet WO 02/092106.

Ces techniques d'enrobage sont bien connues de l'homme de l'art. On peut distinguer les procédés d'enrobage physiques, basés sur la pulvérisation de la solution d'enrobage dans une turbine ou dans un lit fluidisé comme décrit dans les brevets WO 00/30617 et WO 02/092106 d'une part, et l'enrobage physicochimique basé sur la coacervation ou séparation de phases comme décrit dans le brevet US 3341416 d'autre part. Toutes ces techniques conduisent à la mise en place d'une ou plusieurs couches, polymériques externes, recouvrant une particule centrale composée du principe actif pur ou d'un mélange sous forme de granulés de principe actif avec d'autres matériaux supports comme décrit dans le document EPI 194125 de la société Prographarm.

Nous avons vu qu'il n'était pas possible de masquer le goût tout en conservant les propriétés d'absorption de la molécule initiale.

La libération immédiate au niveau du tube digestif, est conditionnée par l'utilisation de polymère pH dépendant très sensible au pH supérieur à 7 de la cavité buccale ou de l'estomac, ce qui implique l'addition d'acide à la formulation finale.

Ces technologies d'enrobage présentent un certain nombre d'inconvénients : la gastroprotection n'est pas totale
le masquage du goût n'est pas total et dans le cas de composés à forte amertume, le goût est encore trop désagréable
- les cinétiques de libération sont modifiées
   les particules d'enrobage ont une taille de quelques centaines de microns, et sont perceptibles lors de l'absorption. Dans ce cas leur rupture peut entraîner un mauvais goût.

Les procédés d'enrobage sont complexes, comportent de nombreuses étapes et ont un coût élevé.

Ces technologies ne sont pas compatibles avec la préparation de sirops stables à long terme

Ces technologies ne sont donc pas encore pleinement satisfaisantes.

De façon totalement surprenante et inattendue, les inventeurs ont montré que l'addition d'acides gras non neutralisés aux compositions des particules lipidiques solides, préparées suivant le procédé décrit dans le brevet WO 99/65448, pouvant contenir un principe actif, permet d'obtenir des particules hydrophobes stables dans l'estomac et qui se libèrent uniquement dans le tube digestif, autorisant ainsi une gastroprotection et un masquage total du goût sans modifier les propriétés de libération de l'actif.

Ainsi la présente invention propose un nouveau système galénique permettant : la gastroprotection,
- le masquage du goût,
   la protection du principe actif, particulièrement en milieu acide,
   la possibilité de modulation des propriétés de libération,
   le masquage des effets d'irritabilité mucosale et de la toxicité de certains principes actifs,
- la préparation de formes aqueuses, à goût masqué, stables et pH indépendantes.

Le système galénique selon l'invention est caractérisé en ce qu'il est constitué par un mélange de composés hydrophobes insolubles dans l'eau, en ce qu'il est sous forme solide à température ambiante et qu'il est totalement dépourvu de composés tensioactifs, de résidus de solvant et d'eau pouvant être à l'origine de réactions d'hydrolyse ou d'oxydation d'un actif qu'il pourrait contenir. Ce système galénique a la capacité d'incorporer des composés de nature hydrophile, hydrophobe ou minérale.

Ainsi, l'invention a pour objet un système galénique, sous forme de particules lipidiques solides, strictement hydrophobe, ne contenant strictement pas d'eau ni de tensioactifs, ni d'agents émulsionnants ni de traces de solvants, caractérisé en ce qu'il comprend au moins une cire hydrophobe et au moins un acide gras non neutralisé.

Par la suite dans le texte, les expressions "système galénique", "particule lipidique", voire encore "gouttelette" ou "gouttelette lipidique" s'entendront comme ayant la même signification.

Selon une forme particulière de l'invention, le système galénique est en outre dépourvu de talc.

Selon encore une autre forme particulière de l'invention, les particules lipidiques sont solides à une température pouvant aller jusqu'à 45 °C, préférentiellement jusqu'à 37,5°C. Selon une autre forme particulière de l'invention, les particules lipidiques sont sous une forme sphérique.

Par cire hydrophobe selon l'invention, on entend que le système galénique peut être constitué d'une ou plusieurs cires, végétales, animales ou minérales, ou d'un mélange d'une ou plusieurs cires et d'au moins une huile non amphiphile

Le système galénique peut en outre comprendre au moins un composé hydrophobe permettant d'ajuster le point de fusion et les propriétés physicochimiques comme la dureté. On peut citer à titre d'exemple de composé hydrophobe, la cire d'abeille ou encore l'huile de palme.

Il convient de choisir une composition appropriée, compatible en termes de toxicité, de biocompatibilité, de non immunogénicité et de biodégradabilité avec l'absorption par voie orale ou, tout autre mode d'administration. Dans ce cas, les composants seront choisis parmi les composants déjà utilisés pour l'administration par voie orale, tels que ceux définis dans la liste GRAS éditée par la Food and Drug Administration, et de telle sorte que les particules formées conservent leurs propriétés d'incorporation, de masquage de goût et de stabilisation des composants actifs.

Ainsi selon l'invention, la cire peut être choisie parmi toute cire connue compatible avec les exigences de l'invention. La cire peut en particulier être choisie parmi les triglycérides et dérivés
l'huile de palme
- la cire de Carnauba
   la cire de Candellila
   la cire d'Alfa
   le beurre de cacao l'ozo érite
   les cires végétales comme la cire d'olivier, la cire de riz, la cire de jojoba hydrogénée ou les cires absolues de fleurs
   les cires d'abeilles et cires d'abeilles modifiées.

Le selon une forme particulière de l'invention, la cire peut être un mélange de cires. Selon l'invention, il est possible d'utiliser une cire ou un mélange de cires, dont le point de fusion peut être compris entre 15°C et 75°C, préférentiellement entre 30°C et 45°C. Selon l'invention, la cire peut être en une quantité comprise entre 0,5% et 99%, préférentiellement entre et 1% et 50%

Selon l'invention, l'acide gras non neutralisé peut être tout acide gras non neutralisé compatible avec les exigences de l'invention. L'acide gras non neutralisé peut être choisi parmi les acides gras à chaînes linéaires ayant un nombre d'atomes de carbone compris entre 4 et 18, tels que par exemple l'acide myristique, l'acide laurique, l'acide palmitique ou encore l'acide oléique.

Selon une forme particulière de l'invention, l'acide gras non neutralisé peut être constitué d'un mélange d'acides gras non neutralisé

Selon l'invention, l'acide gras non neutralisé peut être utilisé à un taux d'acides gras compris entre 0,5% et 75% en masse et préférentiellement entre 1% et 30%.

Le système galénique selon l'invention peut en outre contenir des additifs huileux, pâteux ou solides, des ingrédients actifs liposolubles ou hydrosolubles.

Il est possible d'utiliser d'autres composés, comme les alcools gras de haut poids moléculaire, les acides gras préférentiellement linéaires et saturés pairs ayant de 12 à 30 atomes de carbone, les esters d'acides et d'alcools à haut poids moléculaire notamment les esters des acides linéaires et saturés pairs ayant de 4 à 20 atomes de carbone et des alcools linéaires et saturés pairs ayant de 14 à 32 atomes de carbone. Dans tous les cas le mélange obtenu doit être caractérisé par un point de fusion final compris entre 15°C et 75°C, par l'absence de composés tensioactifs, par un comportement hydrophobe et un non mouillabilité par l'eau. Outre les cires mentionnées ci-dessus, la composition selon l'invention peut contenir une huile ou un mélange choisis parmi :
les huiles de silicones hydrophobes de viscosité comprise entre 5 et 9000 centistockes, les cyclométhicones, les huiles organofluorées lipophiles,
le perhydrosqualène.

D'autres composés huileux comme l'alcool oléique, la lanoline, l'huile de tournesol, l'huile de palme, l'huile d'olive, les acides gras et alcools gras peuvent être utilisés, mais le mélange huileux obtenu doit être caractérisé par un comportement hydrophobe, une absence de miscibilité avec l'eau et un point de fusion compris entre 15°C et 75°C, préférentiellement compris entre 30°C et 45°C.

Il est en outre possible pour ajuster la consistance, d'introduire dans la composition des argiles ou leurs dispersions huileuses, des gommes de silicones phénylées, des amidons, des structurants des corps gras.

On peut ajouter à la matrice hydrophobe du système galénique, un certain nombre de composés comme des charges minérales permettant de moduler la densité et la plasticité. Parmi les composés minéraux, on choisira de façon avantageuse le talc, le kaolin.

Les particules lipidiques selon l'invention peuvent avoir une taille comprise entre

0,5 microns et 1500 microns, préférentiellement entre 10 microns et 250 microns.

Les particules lipidiques selon l'invention présentent l'avantage de permettre la libération retardée d'un actif qu'elles pourraient contenir, une très grande stabilité à pH acide, particulièrement en formulation aqueuse acide, permettant ainsi la protection de l'actif lorsqu'elles sont en contact avec un milieu présentant un pH acide comme par exemple le milieu gastrique, et un masquage total du goût.

L'invention a encore pour objet un système galénique selon l'invention comprenant en outre un actif.

Dans cette description, le terme actif est utilisé pour désigner n'importe quelle substance utilisable en cosmétique, en pharmacie, en biotechnologie, dans le domaine vétérinaire ou encore en alimentation. Particulièrement selon l'invention, l'actif peut être une substance thérapeutique active pouvant être avantageusement administré à l'homme ou aux autres animaux pour diagnostiquer, soigner, réduire, traiter ou prévenir la maladie.

Selon l'invention, l'actif peut être tout composé de nature hydrophile, hydrophobe ou minérale.

Selon l'invention, l'actif peut être dissous ou dispersés dans le système galénique. Bien entendu selon l'invention, l'actif peut être un mélange d'actifs.

L'actif peut être choisi parmi des huiles essentielles, des arômes, des pigments, des charges, des colorants, des enzymes et coenzymes et d'autres substances actives.

Parmi les actifs pouvant être incorporés dans le système galénique selon l'invention, on peut citer les vitamines ou provitamines A, B, C, D, E, PP et leurs esters, les caroténoïdes, les substances anti-radicalaires, les hydroxyacides, les antiseptiques, les molécules agissant sur la pigmentation, sur l'inflammation, les extraits biologiques.

L'actif peut aussi être choisi parmi des conservateurs, des antioxydants, des colorants et pigments, des cellules et organites cellulaires ou encore des composants pharmaceutiques destinés à traiter des pathologies, particulièrement des pathologies cutanées ou mucosales.

A titre d'exemple d'actif thérapeutique pouvant être incorporé dans le système galénique selon l'invention, on peut citer les antibiotiques, les antifongiques, les antiparasitaires, les antipaludéens, les adsorbants, les hormones et dérivés, la nicotine, les antihistaminiques, les anti-inflammatoires stéroïdiens et non stéroïdiens, les agents antiallergiques, les antalgiques, les anesthésiques locaux, les antiviraux, les anticorps et molécules agissant sur le système immunitaire, les cytostatiques et anticancéreux, les antalgiques, les hypolipémiants, les vasodilatateurs, les vasoconstricteurs, les inhibiteurs de l'enzyme de conversion de Pangiotensine et de la phosphodiestérase, les dérivés nitrés et antiangoreux, les béta-bloquants, les inhibiteurs calciques, les antidiurétiques et diurétiques, les bronchodilatateurs, les opiacés et dérivés, les barbituriques, les benzodiazépines, les molécules agissant sur le système nerveux central, les acides nucléiques, les peptides, les composés anthracéniques, l'huile de paraffine, le polyéthylène glycol, les sels minéraux, les antispasmodiques, les antisécrétoires gastriques, les pansements gastriques argiles et polyvinylpyrrolidone, l'amidon. Cette liste exhaustive n'est en aucun cas limitative.

Selon l'invention, les particules lipidiques ayant une taille comprise entre 0,5 microns et 1500 microns, un point de fusion compris entre 15°C et 75°C, strictement hydrophobe, ne contenant strictement pas d'eau, ni de tensioactifs, ni d'agents émulsionnants, ni de traces de solvants, comprenant en outre un actif au moins une cire hydrophobe et au moins un acide gras non neutralisé, tel que l'actif est absent de la surfaces des particules lipidiques. La température de fusion, après incorporation du principe actif est préférentiellement comprise entre 30 et 45°C.

La capacité de chargement des particules en actif peut s'étendre de 0,02 % à 75 % par rapport au poids de particules, particulièrement de 5% à 50%.

L'homme de l'art sait que lorsqu'on effectue l'incorporation de ces actifs dans le système galénique, il convient de choisir une composition lipidique appropriée de telle sorte que les particules soient solides à la température d'utilisation avec une taille comprise de préférence entre 0,5 microns et 1500 microns et de préférence entre 0,5 microns et 100 microns, permettent d'obtenir un masquage total du goût sans modifier les propriétés de libération et en présentant une très grande stabilité en formulation aqueuse même à pH élevé. En outre, il est nécessaire que le procédé de préparation dudit système galénique comprenant en outre un actif puisse être mis en oeuvre.

Selon l'invention, le système galénique comprenant en outre un actif qui peut être préparé suivant le procédé décrit dans le brevet WO 99/65448.

Selon ce mode de réalisation du procédé, les particules sont obtenues par mélange à chauffage modéré. Plus précisément ces compositions sont obtenues par un procédé caractérisé par le fait que l'on mélange à la température de fusion de la cire, la cire et l'huile jusqu'à obtenir un mélange caractérisé par une température de fusion inférieure à la température de fusion de la cire. Le rapport initial entre la cire et l'huile peut être modulé pour que la température de fusion du mélange final soit inférieure à la température de dégradation du composé à incorporer, le plus sensible à la chaleur. Le mélange final doit être solide à température d'utilisation et doit présenter dans une de ces mises en forme préférentielles un point de fusion de 37,5°C. Le mélange est ensuite refroidi sous agitation adaptée, à une température supérieure de 2°C ou 3°C à son point de fusion, pour permettre l'inclusion des actifs pharmaceutiques. Le mélange est alors mis en forme pour donner des particules sphériques hydrophobes appelées particules.

En comparaison avec les techniques de fusion chaude, le procédé de la présente invention, ne fait pas intervenir d'agents émulsionnants ni de produits amphiphiles dans la composition, pour permettre une dispersion stable lors de la phase de solidification par refroidissement.

Selon un mode de réalisation particulier du procédé selon l'invention, quand il est nécessaire d'assurer une élimination totale de l'actif de la surface des particules lipidiques, celui-ci comprend une étape de lavage desdites particules obtenues avec un mélange de lavage comprenant de l'éthanol. Dans ce cas, la présence d'éthanol dans le mélange de lavage est essentielle au procédé car l'éthanol permet un lavage complet des éventuels résidus d'actif pouvant être présents à la surface des particules lipidiques qui pourraient générer un goût désagréable.

Ainsi selon cette mise en oeuvre particulière du procédé selon l'invention, le mélange des différents composants du système galénique (la cire et l'acide gras non neutralisé entre autres) et de l'actif est réalisé dans une première étape du procédé. Ce mélange est réalisé à chaud à 2°C ou 3°C au-dessus du point de fusion du composé présentant le point de fusion le plus élevé. L'homme de l'art sait qu'il est nécessaire d'appliquer un mode d'agitation approprié à la dispersion de tous les composants.

Puis dans une deuxième étape on forme des gouttelettes lipidiques comprenant l'actif en dispersant le mélange obtenu à la première étape dans un gel préparé avec un agent gélifiant, rhéofluidifiant et non tensioactif, avec lequel ledit mélange est non miscible, préalablement porté à la même température, et de concentration en agent gélifiant comprise entre 0,1 g/1 et 30 g/1, préférentiellement comprise entre 0,2 g/1 et 20 g/1 suffisamment élevée pour figer la dispersion.

Il peut être préférable d'injecter la composition au sein du gel, par exemple par un orifice situé à la base d'un réacteur. L'agitation qui doit être maintenue tout au long de l'injection a pour caractéristique de présenter une pale équipée d'une ancre, destinée à disperser la composition et une deuxième pale axiale équipée d'une hélice tripale destinée à former les gouttelettes de dispersion de taille souhaitée. Cette dernière étape est extrêmement rapide puisque les gouttelettes sont obtenues au fur et à mesure de l'injection de la composition. L'agitation n'a pas besoin d'être maintenue après la fin de l'injection, car les gouttelettes sont figées dans le gel.

Dans une troisième étape du procédé, dès la fin de l'injection, les gouttelettes sont refroidies immédiatement, sous la température de solidification du mélange, puis lavées avec un mélange de lavage contenant de l'eau et entre 0% à 25% d'éthanol. La phase de lavage est très importante car elle permet de ne pas avoir de résidus à la surface des particules, qui pourraient générer un goût désagréable.

Ainsi, quelle que soit la forme de mise en oeuvre du procédé de l'invention, le lavage des particules peut être réalisé en utilisant un mélange de lavage constitué d'eau et d'éthanol en une proportion comprise entre 1 % et 10% d'éthanol.

Enfin dans une quatrième étape, les particules lavées sont ensuite récupérées par tamisage puis séchées. Les particules obtenues présentent une grande homogénéité de taille et peuvent être manipulées industriellement sans précaution particulière.

L'homme de l'art sait qu'on peut aussi utiliser d'autres modes de dispersion comme la sonication ou les mélangeurs statiques.

Le procédé selon l'invention est donc rapide et ne nécessite pas d'étape d'agitation longue et délicate. Il permet d'incorporer l'actif au système galénique dès la première étape du mélange des différents ingrédients de la composition.

Parmi les agents gélifiants rhéofluidifiants et non tensioactifs appropriés pour la formation du gel utilisé comme milieu de dispersion selon le procédé, on peut citer les polymères carboxyvinyliques tels que les polymères polyacryliques non modifiés par des groupements hydrophobes ou surfactants, les carraghénanes, les épaississants et gélifiants polysaccharidiques comme les xanthanes, les gommes de guar et de caroube, les alginates, les dérivés de cellulose, les pectines, l'agar, ou un mélange de ceux-ci.

Les particules lipidiques comprenant un actif peuvent être incorporées dans toute composition, particulièrement dans toute composition, cosmétique, pharmaceutique, vétérinaire ou alimentaire.

Ainsi l'invention a aussi pour objet une composition comprenant au moins une particule lipidique contenant un actif.

La composition selon l'invention, peut en outre comprendre tout additif visant à en modifier l'aspect ou la rhéologie. Par exemple, à la poudre sèche de particules, on peut ajouter des agents lubrifiants qui améliorent la fluidité des particules comme le talc, les amidons, les poudres de silice, les agents antistatiques. Bien entendu la composition selon l'invention peut être sous la forme de toute formulation galénique adéquate. Dans une mise en forme avantageuse de réalisation, les particules de l'invention sont mises en oeuvre au sein de suspensions aqueuses, sirops et sachets. Enfin les particules peuvent être mises en oeuvre au sein de formulations galéniques classiques du type capsules, gélules, granulés, poudres orales, poudres dispersibles, comprimés, comprimés hydrodispersibles et orodispersibles.

Selon un autre aspect de l'invention, les compositions peuvent être utilisées pour l'administration par voie injectable et en particulier pour la préparation de formes à libération prolongée. Dans ce cas, les particules lipidiques selon l'invention, sont préparées pour avoir une taille comprise préférentiellement entre 0,5 µm et 5 µm. Il est préférable de les tamiser pour obtenir une distribution de taille conforme avec le mode d'administration. Leur composition cireuse est choisie pour être conforme avec les nécessités de la voie injectable. Cette forme galénique permet d'éliminer les problèmes de toxicité rencontrés par les particules polymériques obtenues avec les procédés de polymérisation en émulsion, liés à l'utilisation des solvants et des composés tensioactifs. Les particules selon l'invention permettent d'obtenir des taux de chargement en principe actif compris entre 0,10 et 2 grammes/gramme de matrice cireuse. L'homme de l'art sait que les technologies d'encapsulation ne permettent pas d'atteindre ces taux.

Enfin la dégradation des particules n'entraîne pas de réaction inflammatoire comme on peut le rencontrer avec les particules injectables à base de polymère de polylactique-glycolique.

Les exemples qui suivent servent seulement à illustrer l'invention. Pour certains des exemples suivants, les tests de masquage de goût ont été réalisés auprès d'un échantillon de 10 individus. Les résultats sont exprimés selon l'échelle suivante :
1 : le goût du principe actif n'est pas détecté
2 : le goût du principe actif est légèrement perçu
3 : le goût du principe actif est détecté
4 : le goût du principe actif est encore acceptable
5 : le goût du principe actif n'est pas acceptable

### Exemple 1 : Particules contenant de l'érythromycine

Exemple donné pour la fabrication de 120 g de particules contenant de l'érythromycine : Composition :
- Huile de palme 80 g
- Acide oléique 5 g
- Acide stéarique 4 g
- Trilaurine 1 g
- Erythromycine 30 g
soit 120 g de particules sèches contiennent 30 g d'érythromycine

Mode opératoire :
Dans un récipient thermostaté, on porte le composé de plus haut point de fusion,
2°C au-dessus de sa température de fusion, puis on ajoute progressivement les différents composés du point de fusion le plus élevé au moins élevé. La température du mélange est progressivement abaissée pour être maintenue 2°C à 3°C au-dessus de la température de fusion du nouveau mélange obtenu. On ajoute en dernier l'érythromycine. La dispersion de ces composants, dans la phase lipidique, est réalisée à l'aide d'un système d'agitation équipé d'un mobile en forme d'ancre à une vitesse de 200 tours/min. Lorsque le mélange est homogène, il est ajouté à 600 ml de gel aqueux à 0,2 % de carbopol Ultrez 10, neutralisé à pH 6,5 avec de la soude, préalablement porté à la même température que le mélange lipidique et contenu dans un réacteur équipé d'un système d'agitation à hélice tripale. Pendant l'addition de la composition, la vitesse d'agitation de l'hélice tripale est de 110 tours/min. L'agitation est maintenue pendant 30 secondes après la fin de l'addition de la composition, puis stoppée. La dispersion est alors refroidie à 15°C. Les particules sont récupérées par, puis lavées à l'eau distillée, puis par un mélange d'eau distillée à 15% d'éthanol, puis récupérées et séchées. Les particules ainsi obtenues ont une taille moyenne de 62 microns. Ces particules sont totalement dépourvues d'actif à leur surface. Sur les particules, après extraction, on réalise un dosage de l'érythromycine par HPLC. On obtient 29,3 g d'érythromycine pour 100 g de matrice
H est bien connu que les compositions contenant de l'érythromycine présentent un goût prononcé. Le test de masquage de goût réalisé sur les particules obtenues ne donne aucune détection du principe actif.

### Exemple 2 : Préparation d'un sirop contenant des particules chargées en érythromycine

Avec un sirop de saccharose pharmaceutique, distribué par la société Cooper, dénommé Sirop Simple de composition :
- Saccharose 86,50 g
- Parahydroxybenzoate de méthyle sodique 0, 15 g
- Parahydroxybenzoate de propyle sodique 0,03 g
- Eau purifiée qsp 100 ml

A 250 ml de sirop, à température ambiante, on incorpore 20 g de particules chargées en érythromycine, obtenues selon l'exemple 1, ce qui correspond à 5,86 g d'érythromycine. Le test de goût réalisé sur le sirop ne donne aucune détection du principe actif.

### Exemple 3 : Préparation d'une poudre pour la voie orale hydrodispersible, contenant des particules chargées en érythromycine

Dans un mélangeur à poudre, on place :
Particules selon l'exemple 1100 g
   - Arôme 7 g
      Aspartame 3 g

Gomme de xanthane 1 g Après mélange, la poudre est répartie en sachet unitaire de 2,24 g contenant 500 mg d'érythromycine. La reprise par 50 ml d'eau permet de reconstituer une dispersion aqueuse de l'antibiotique. Le test de goût réalisé sur la dispersion ne donne aucune détection du principe actif.

### Exemple 4 : Particules contenant du paracétamol

Exemple donné pour la fabrication de 100 g de particules permettant de diminuer la gastrotoxicité du paracétamol :
Composition :
   - Huile de palme 49,0 g
   - Acide oléique 20,0 g
   - Acide stéarique 4,5 g
   - Acide caprique 1,0 g
   - Acide béhénique 0,5 g
   - Paracétamol 25 g

Le mode opératoire est identique à celui décrit dans l'exemple 1.

Le test de masquage de goût réalisé sur les particules ne donne aucune détection du principe actif.

### Exemple 5 : Particules contenant de l'oxytétracycline

Exemple donné pour la préparation de 100 g particules injectables à libération contrôlée contenant de l'oxytétracycline :
Composition :
   - Trilaurine 39 g
   - Tricaprine 32 g
   - Acide oléique 3 g
   - Acide stéarique 1 g
   - Oxytétracycline 25 g

Comme indiqué à l'exemple 1, les particules sont obtenues par la dispersion de la phase lipidique dans la phase aqueuse gélifiée sous agitation. La concentration en carbopol de la phase aqueuse est de 0,05 %. Pour diminuer la taille moyenne des particules à 1 µm, l'agitation est réalisée à l'aide d'une tige à turboagitation axiale, à une vitesse de 300 tours/min. L'agitation est maintenue pendant 60 secondes après la fin de l'addition de la composition, puis stoppée. La dispersion est alors refroidie à 15°C. Les particules sont récupérées par tamisage, puis lavées à l'eau distillée, puis par un mélange d'eau distillée à 15% d'éthanol, puis récupérées et séchées. Les particules ainsi obtenues ont une taille moyenne de 1,2 microns.

## Revendications

1. Système galénique, sous forme de particules lipidiques solides ayant une taille comprise entre 0,5 microns et 1500 microns, un point de fusion compris entre 15°C et 752°C, strictement hydrophobe, ne contenant strictement pas d'eau, ni de tensioactifs, ni d'agents émulsionnants, ni de traces de solvants, comprenant au moins une cire hydrophobe **caractérisé en ce qu'**il comprend au moins un acide gras non neutralisé et au moins un actif tel que l'actif est absent de la surface des particules lipidiques.

2. Système galénique selon la revendication 1, **caractérisé en ce qu'**il est solide à une température pouvant aller jusqu'à 45°C, préférentiellement jusqu'à 37, 5°C.

3. Système galénique selon l'une quelconque des revendications 1 ou 2, **caractérisé en ce que** les particules lipidiques sont sous une forme sphérique.

4. Système galénique selon l'une quelconque des revendications 1 à 3, **caractérisé en ce que** la cire hydrophobe est une cire végétale, animale ou minérale, ou un mélange d'au moins une cire et d'au moins une huile non amphiphile.

5. Système galénique selon l'une quelconque des revendications 1 à 4, **caractérisé en ce que** la cire est en une quantité comprise entre 0,5% et 99%, préférentiellement entre 1% et 55%.

6. Système galénique selon l'une quelconque des revendications 1 à 5, **caractérisé en ce qu'**il comprend en outre au moins un composé hydrophobe.

7. Système galénique selon l'une quelconque des revendications 1 à 6, **caractérisé en ce que** la cire a un point de fusion compris entre 15°C et 75°C, préférentiellement entre 30°C et 452°C.

8. Système galénique selon l'une quelconque des revendications 1 à 7, **caractérisé en ce que** la cire est choisie parmi les triglycérides et dérivés, l'huile de palme, la cire de Carnauba, la cire de Candellila, la cire d'Alfa, le beurre de cacao, l'ozokérite, les cires végétales comme la cire d'olivier, la cire de riz, la cire de jojoba hydrogénée ou les cires absolues de fleurs, les cires d'abeilles ou les cires d'abeilles modifiées.

9. Système galénique selon l'une quelconque des revendications 1 à 8, **caractérisé en ce que** l'acide gras non neutralisé est choisi parmi les acides gras à chaînes linéaires ayant un nombre d'atome de carbone compris entre 4 et 18, tels que par exemple l'acide myristique, l'acide laurique, l'acide palmitique ou encore l'acide oléique.

10. Système galénique selon l'une quelconque des revendications 1 à 9, **caractérisé en ce que** l'acide gras est un taux d'acides gras compris entre 0,5% et 75% en masse et préférentiellement entre 1% et 30%

11. Système galénique selon l'une quelconque des revendications 1 à 10, **caractérisé en ce qu'**il se présente sous la forme de particules lipidique ayant une taille comprise entre 10 microns et 250 microns.

12. Système galénique selon l'une quelconque des revendications 1 à 11, **caractérisé en ce qu'**il a une température de fusion comprise entre 30°C et 45°C.

13. Système galénique selon l'une quelconque des revendications 1 à 12, **caractérisé en ce que** capacité de chargement des particules en actif peut s'étendre de 0,02 % à 75 % par rapport au poids de particules, particulièrement de 5% à 50%.

14. Procédé de préparation d'un système galénique sous forme de particules lipidiques solides ayant une taille comprise entre 0,5 microns et 1500 microns, un point de fusion compris entre 15°C et 75°C, strictement hydrophobe, ne contenant strictement pas d'eau, ni de tensioactifs, ni d'agents émulsionnants, ni de traces de solvants, comprenant un actif, au moins une cire hydrophobe et au moins un acide gras non neutralisé, tel que l'actif est absent de la surface des particules lipidiques, comprenant les étapes suivantes :
- dans une première étape, on mélange au moins une cire hydrophobe et au moins un acide gras non neutralisé, sous agitation, à chaud à 2°C ou 3°C au-dessus du point de fusion du composé présentant le point de fusion le plus élevé, puis on ajoute l'actif,
- dans une deuxième étape, on forme des gouttelettes lipidiques comprenant l'actif en dispersant le mélange obtenu à la première étape dans un gel avec lequel ledit mélange est non-miscible, préalablement porté à la même température que le mélange obtenu à la première étape, et de concentration en agent gélifiant comprise entre 0,1 g/l et 30 g/l, de préférence entre 0,2 g/l et 20 g/l,
- dans une troisième étape, dès la fin de la dispersion, les gouttelettes sont refroidies immédiatement sous la température de solidification du mélange, puis lavées avec un mélange de lavage contenant de l'eau et entre 0% à 25% d'éthanol,
- dans une quatrième étape, les particules lavées ayant une taille comprise entre 0,5 microns et 1500 microns sont récupérées par tamisage puis séchées.

15. Procédé selon la revendication 14, **caractérisé en ce que** dans le mélange de lavage, l'éthanol est en une proportion comprise entre 1% et 10%.

16. Procédé selon l'une quelconque des revendications 14 ou 15, dans lequel le gel est préparé avec un agent gélifiant, rhéofluidifiant et non tensioactif, choisi parmi les polymères carboxyvinyliques tels que les polymères polyacryliques non modifiés par des groupements hydrophobes ou tensioactifs, les carraghénanes, les épaississants et gélifiants polysaccharidiques comme les xanthanes, les gommes de guar et de caroube, les alginates, les dérivés de cellulose, les pectines, l'agar, ou un mélange de ceux-ci.

17. Système galénique sous forme de particules lipidiques solides comprenant un actif, susceptible d'être obtenues par le procédé selon l'une des revendications 14-16.

18. Composition comprenant au moins un système galénique contenant un actif selon l'une des revendications 1 à 13 et 17.

19. Composition selon la revendication 18, **caractérisée en ce qu'**elle est une composition cosmétique, pharmaceutique, vétérinaire ou alimentaire.

20. Composition selon l'une quelconque des revendications 18 ou 19, destinée à être utilisée pour une administration par voie orale ou par injection.

## Patentansprüche

1. Galenisches System, in der Form von festen Lipidpartikeln mit einer Größe im Bereich zwischen 0,5 Mikron und 1500 Mikron, einem Schmelzpunkt im Bereich zwischen 15 °C und 75 °C, streng hydroph ob, streng weder Wasser, noch Tenside, noch Emulgatoren, noch Spuren von Lösungsmitteln enthaltend, mindestens ein hydrophobes Wachs umfassend, **dadurch gekennzeichnet, dass** es mindestens eine nicht neutralisierte Fettsäure und mindestens einen Wirkstoff umfasst, so dass der Wirkstoff von der Oberfläche der Lipidpartikel abwesend ist.

2. Galenisches System nach Anspruch 1, **dadurch gekennzeichnet, dass** es bei einer Temperatur fest ist, die bis zu 45 °C, vorzugsweise bis zu 37,5 °C reichen kann.

3. Galenisches System nach einem der Ansprüche 1 oder 2, **dadurch gekennzeichnet, dass** die Lipidpartikel kugelförmig sind.

4. Galenisches System nach einem der Ansprüche 1 bis 3, **dadurch gekennzeichnet, dass** das hydrophobe Wachs ein pflanzliches, tierisches oder mineralisches Wachs oder ein Gemisch aus mindestens einem Wachs und mindestens einem nicht amphiphilen Öl ist.

5. Galenisches System nach einem der Ansprüche 1 bis 4, **dadurch gekennzeichnet, dass** das Wachs in einer Menge im Bereich zwischen 0,5 % und 99 %, vorzugsweise zwischen 1 % und 55 % vorhanden ist.

6. Galenisches System nach einem der Ansprüche 1 bis 5, **dadurch gekennzeichnet, dass** es ferner mindestens eine hydrophobe Verbindung umfasst.

7. Galenisches System nach einem der Ansprüche 1 bis 6, **dadurch gekennzeichnet, dass** das Wachs einen Schmelzpunkt im Bereich zwischen 15 °C und 75 °C, vorzugsweise zwischen 30 °C und 45 °C hat.

8. Galenisches System nach einem der Ansprüche 1 bis 7, **dadurch gekennzeichnet, dass** das Wachs ausgewählt ist aus den Triglyceriden und Derivaten, Palmöl, Carnaubawachs, Candelillawachs, Alfagraswachs, Kakaobutter, Ozokerit, aus pflanzlichen Wachsen wie Olivenbaumwachs, Reiswachs, hydriertes Jojobawachs oder aus den Absolue-Blütenwachsen, Bienenwachsen oder modifizierten Bienenwachsen.

9. Galenisches System nach einem der Ansprüche 1 bis 8, **dadurch gekennzeichnet, dass** die nicht neutralisierte Fettsäure ausgewählt ist aus den geradkettigen Fettsäuren mit einer Anzahl von Kohlenstoffatomen im Bereich zwischen 4 und 18, wie zum Beispiel Myristinsäure, Laurinsäure, Palmitinsäure oder auch Ölsäure,

10. Galenisches System nach einem der Ansprüche 1 bis 9, **dadurch gekennzeichnet, dass** die Fettsäure in einem Fettsäuregehalt im Bereich zwischen 0,5 Gew.-% und 75 Gew.-% und vorzugsweise zwischen 1 Gew.-% und 30 Gew.-% vorhanden ist

11. Galenisches System nach einem der Ansprüche 1 bis 10, **dadurch gekennzeichnet, dass** es in der Form von Lipidpartikeln mit einer Größe im Bereich zwischen 10 und 250 Mikron vorliegt.

12. Galenisches System nach einem der Ansprüche 1 bis 11, **dadurch gekennzeichnet, dass** es eine Schmelztemperatur im Bereich zwischen 30 °C und 45 °C aufweist.

13. Galenisches System nach einem der Ansprüche 1 bis 12, **dadurch gekennzeichnet, dass** sich die Wirkstoffbeladungskapazität der Partikel, bezogen auf das Gewicht der Partikel, von 0,02 % bis 75 %, insbesondere von 5 % bis 50 % erstrecken kann.

14. Verfahren zum Herstellen eines galenischen Systems in der Form von festen Lipidpartikeln, mit einer Größe im Bereich zwischen 0,5 Mikron und 1500 Mikron, einem Schmelzpunkt im Bereich zwischen 15 °C und 75 °C, streng hydrophob, streng weder Wasser, noch Tenside, noch Emulgatoren, noch Spuren von Lösungsmitteln enthaltend, einen Wirkstoff umfassend, mindestens ein hydrophobes Wachs und mindestens eine nicht neutralisierte Fettsäure, so dass der Wirkstoff von der Oberfläche der Lipidpartikel abwesend ist, folgende Schritte umfassend:
- in einem ersten Schritt erfolgt das Mischen mindestens eines hydrophoben Wachses und mindestens einer nicht neutralisierten Fettsäure, unter Rühren, erhitzt zu 2 °C oder 3 °C über den Sc hmelzpunkt der Verbindung, die den höchsten Schmelzpunkt aufweist, dann erfolgt die Zugabe des Wirkstoffs,
- in einem zweiten Schritt erfolgt die Bildung der Lipidtröpfchen, die den Wirkstoff umfassen, durch Dispergieren des im ersten Schritt erhaltenen Gemischs in ein Gel, mit dem das Gemisch nicht mischbar ist, zuvor auf die gleiche Temperatur gebracht wie die des im ersten Schritt erhaltenen Gemischs, und einer Geliermittelkonzentration im Bereich zwischen 0,1 g/l und 30 g/l, vorzugsweise zwischen 0,2 g/l und 20 g/l,
- in einem dritten Schritt, nach Ende der Dispersion, werden die Tröpfchen sofort unter die Erstarrungstemperatur des Gemischs abgekühlt, anschließend mit einem Waschgemisch, das Wasser und Ethanol in einer Menge zwischen 0 % und 25 % enthält, gewaschen,
- in einem vierten Schritt werden die gewaschenen Partikel mit einer Größe im Bereich zwischen 0,5 Mikron und 1500 Mikron durch Sieben wiedergewonnen und anschließend getrocknet.

15. Verfahren nach Anspruch 14, **dadurch gekennzeichnet, dass** das Ethanol im Waschgemisch in einem Mengenverhältnis im Bereich zwischen 1 % und 10 % vorhanden ist.

16. Verfahren nach einem der Ansprüche 14 oder 15, wobei das Gel mit einem strukturviskosen und nicht tensidischen Geliermittel zubereitet wird, ausgewählt aus Carboxyvinylpolymeren wie nicht durch hydrophobe oder tensioaktive Gruppen modifizierte Polyacrylpolymere, Carrageenane, Polysaccharid-Verdickungsmittel und -Geliermittel wie Xanthane, Guar- und Johannisbrotgummi, Alginate, Cellulosederivate, Pektine, Agar oder einem Gemisch davon.

17. Galenisches System in der Form von festen Lipidpartikeln, einen Wirkstoff umfassend, die mittels des Verfahrens nach einem der Ansprüche 14-16 erhalten werden können.

18. Zusammensetzung, umfassend mindestens ein galenisches System, das einen Wirkstoff nach einem der Ansprüche 1 bis 13 und 17 enthält.

19. Zusammensetzung nach Anspruch 18, **dadurch gekennzeichnet, dass** sie eine kosmetische, pharmazeutische, veterinäre Zusammensetzung oder eine Nahrungsmittelzusammensetzung ist.

20. Zusammensetzung nach einem der Ansprüche 18 oder 19, vorgesehen zum Anwenden für eine Verabreichung auf oralem Wege oder durch Injektion.

## Claims

1. Galenic system in the form of solid lipid particles between 0.5 microns and 1500 microns in size with a melting point between 15°C and 75°C, strictly hydrophobic, strictly containing no water, surfactants, emulsifying agents, or solvents traces, comprising at least one hydrophobic wax **characterised in that** it is composed of at least one non-neutralized fatty acid and at least one substance so that this substance is absent from the surface of the lipid particles.

2. Galenic system according to claim 1, **characterised by** being solid at a temperature of up to 45°C, preferably up to 37.5°C.

3. Galenic system according to either or both of claims 1 or 2, **characterised by** the lipid particles being spherical in shape.

4. Galenic system according to any of claims 1 to 3, **characterised in that** the hydrophobic wax is a vegetable, animal, or mineral wax, or a mixture of at least one wax and at least one non-amphiphilic oil.

5. Galenic system according to any of claims 1 to 4, **characterised in that** the wax is in an amount between 0.5% and 99%, preferentially between 1% and 55%.

6. Galenic system according to any of claims 1 to 5, **characterised by** it further comprising at least one hydrophobic compound.

7. Galenic system according to any of claims 1 to 6 **characterised in that** the wax has a melting point between 15°C and 75°C, preferably between 30°C and 45°C.

8. Galenic system according to any of claims 1 to 7, **characterised by** the wax being chosen from triglycerides and derivatives, palm oil, carnauba wax, candelilla wax, alfa wax, and cocoa butter, ozokerite, vegetable waxes such as olive wax, rice wax, hydrogenated jojoba wax, absolute flower waxes, beeswax, or modified beeswaxes.

9. Galenic system according to any of claims 1 to 8 **characterised by** the non-neutralized fatty acid being selected from branched chain fatty acids with 4 to 18 carbon atoms such as myristic, lauric, palmitic, or oleic acids.

10. Galenic system according to any of claims 1 to 9 **characterised by** the fatty acid is a fatty acid rate between 0.5% and 75% by weight and preferably between 1 % and 30%.

11. Galenic system according to any of claims 1 to 10 **characterised by** it being in the form of lipid particles 10 to 250 microns in size.

12. Galenic system according to any of claims 1 to 11, **characterised by** it having a melting point between 30°C and 45°C.

13. Galenic system according to any of claims 1 to 12 **characterised by** the loading capacity of active particles ranging from 0.02% to 75% by weight of particles, particularly from 5% to 50%.

14. Method of preparing a galenic system in the form of solid lipid particles between 0.5 and 1500 microns in size, a melting point between 15°C and 75°C, and strictly hydrophobic, strictly c ontaining no water, no surfactants, no emulsifiers, no solvents traces, comprising a substance, at least a hydrophobic wax and at least one non-neutralized fatty acid so that the substance is absent from the surface of the lipid particles, including the following steps:
- In the first step, at least one hydrophobic wax and at least one non neutralized fatty acid are mixed and stirred under heat at 2°C or 3°C above the melting point of the compound presenting the highest melting point, and then the substance is added.
- In the second stage, lipid droplets composed of the substance are formed by dispersing the mixture obtained in the first step into a gel with which this mixture is immiscible, brought beforehand to the same temperature as the mixture obtained at the first stage, the concentration of gelling agent is between 0.1 g/l and 30 g/l, preferably between 0.2 g/l and 20 g/l.
- In the third step, at the end of the dispersion, the droplets are immediately cooled below the temperature of mixture solidification, then washed with a washing mixture containing water and between 0% to 25% ethanol.
- In the fourth step, the washed particles between 0.5 and 1500 microns in size are collected by sieving and then dried.

15. Method according to claim 14 **characterised by** ethanol in the washing mixture being in a proportion of 1 % to 10%.

16. Method according to either or both of claims 14 or 15 in which the gel is prepared with a gelling, shear-thinning, and non-surfactant agent selected from carboxyvinyl polymers such as polyacrylic polymers not modified by hydrophobic or surfactant groups, carrageenans, thickeners, polysaccharide gelling agents such as xanthones, guar gum, and locust bean gum, alginates, cellulose derivatives, pectins, agar, or a mixture of these.

17. Galenic system in the form of solid lipid particles composed of a substance that may be obtained by the method according to claims 14-16.

18. Composition consisting of at least one galenic system containing a substance according to any of claims 1 to 13 and 17.

19. Composition according to claim 18, **characterised by** it being a cosmetic, pharmaceutical, veterinary, or food composition.

20. Composition according to either or both of claims 18 or 19, intended for oral use or injected.
